# EUROPEAN PATENT SPECIFICATION

(11) **EP 3 709 016 B1**
(45) Date of publication and mention of the grant of the patent: **20.12.2023**
(21) Application number: 20158454.7
(22) Date of filing: 20.02.2020
(51) Int. Cl.: G01N 33/50, C12Q 1/02

(54) **METHOD FOR CARRYING OUT AN IN VITRO CYTOTOXICITY TEST ON RETINAL TISSUE**
METHODE ZUR DURCHFÜHRUNG EINES IN VITRO ZYTOTOXIZITÄTSTESTS AN NETZHAUTGEWEBE
METHODE POUR LA REALISATION D'UN TEST DE CYTOTOXICITÉ SUR DU TISSU DE RÉTINE

(30) Priority: 12.03.2019 IT 201900003605
(43) Date of publication of application: 16.09.2020
(73) Proprietor: AL.CHI.MI.A. S.r.l., 35020 Ponte S. Nicolò (Padova) (IT)
(72) Inventor: Beccaro, Mauro, 35010 Cadoneghe (Padova) (IT); Bettini, Enrico, 30032 Fiesso D'Artico (Venezia) (IT); Signori, Paolo, 37100 Verona (IT); Gatto, Claudio, 31050 Ponzano Veneto (TV) (IT)
(74) Representative: Ponchiroli, Simone

(56) References cited:
- WO-A1-2017/207845
- WO-A2-03/058251
- US-A- 5 928 860
- International Organization For Standardization: "ISO 10993-5 Biological evaluation of medical devices - Part 5: Tests for in vitro cytotoxicity", , 6 November 2009 (2009-11-06), XP055647777, Retrieved from the Internet: URL:http://nhiso.com/wp-content/uploads/20 18/05/ISO-10993-5-2009.pdf [retrieved on 2019-11-29]
- SAMANTHA L. WILSON ET AL: "An overview of current techniques for ocular toxicity testing", TOXICOLOGY., vol. 327, 1 January 2015 (2015-01-01), pages 32-46, XP055562651, IR ISSN: 0300-483X, DOI: 10.1016/j.tox.2014.11.003

## Description

This invention relates to a method for carrying out an in vitro cytotoxicity test in the ophthalmic sector, and has been devised in particular for the purpose of identifying a reliable method for verifying the cytotoxicity or non-cytotoxicity of all of the substances which may be used in ophthalmic treatments in general, but above all in ophthalmic surgery.

It should be noticed that in the context of this invention the definition of substance is intended to indicate pure substances, mixtures or solutions, compounds or compositions, as well as all substances which are usually classed as medical devices.

For example, this invention is intended for ascertaining the cytotoxicity or non-cytotoxicity of medical devices such as endotamponades, for example perfluorocarbons or silicone oils, dyes, and infusion fluids.

At present, the cytotoxicity test commonly adopted in this sector, is an in vitro test governed by ISO 10993-5, which provides for testing substances using cultured cells.

The toxicity is evaluated after a period of incubation of the cultured cells in contact with a predetermined concentration of the substance to be tested, and the evaluation is usually carried out using vital dyes which are capable of discriminating between living and dead cells (selectively staining either one or the other).

As required by the above-mentioned ISO standard, for medical devices in the ophthalmic sector the cultured cells to be used must be cultured cells derived from the human retinal pigmented epithelium, such as that marketed as ARPE-19 (ATCC^{®} CRL-2302^{™}).

However, recent clinical trials have shown that these in vitro cytotoxicity tests, if not conducted in an appropriate way, may provide unreliable results, failing to highlight the cytotoxicity of some substances which, used in ophthalmic surgery, in contrast caused serious harm to the patients operated on (see Pastor JC, Coco RM, Fernandez-Bueno I, Alonso-Alonso ML, Medina J, Sanz-Arranz A et al. ACUTE RETINAL DAMAGE AFTER USING A TOXIC PERFLUORO-OCTANE FOR VITREO-RETINAL SURGERY. Retina 2017 Jun;37(6):1140-1151). The authors of those trials therefore highlighted the need to find new biological methods for testing the safety of substances to be used in the ophthalmic sector. Another toxicity test is disclosed in US5928860A, where a tissue sample is placed in a gradient perfusion chamber. However, this is a relatively complex setup.

In this context, the technical purpose which forms the basis of this invention has been to provide a new method for carrying out an in vitro cytotoxicity test, in the ophthalmic sector, which overcomes the above-mentioned disadvantages.

In particular, the technical purpose of this invention has been to provide a method for carrying out an in vitro cytotoxicity test in the ophthalmic sector which guarantees greater reliability than the prior art methods.

The technical purpose specified and the aims indicated are substantially achieved by a method for carrying out an in vitro cytotoxicity test in the ophthalmic sector as described in the appended claims.

Further features and the advantages of this invention are more apparent in the detailed description which follows, of several preferred, non-limiting embodiments of a method for carrying out an in vitro cytotoxicity test in the ophthalmic sector. What is described below must always be understood to refer not only to a specific substance to be tested but also to a specific concentration thereof.

A first innovative aspect which forms the basis of this invention consists of having identified the human retina explanted from a deceased subject, and considered in its full thickness (that is to say, including all of the layers of which it is composed), as a more reliable alternative to the cell culture derived from the human retinal pigmented epithelium. Indeed, tests carried out by the Applicant have shown how the different retinal layers used together in conditions similar to physiological conditions, may respond very differently to the same substance than only retinal pigmented epithelial cells, and how an apparently non-cytotoxic substance if tested only on retinal pigmented epithelial cells, may in contrast also have extremely toxic effects on the cells of other retinal layers.

A second innovative aspect which forms the basis of this invention consists of having provided a sequence of steps for carrying out a cytotoxicity test on an explanted human retina.

A further innovative aspect which forms the basis of this invention consists of having identified the ways and timing for managing the explanting of the human retina from a deceased subject and its management for carrying out the cytotoxicity test.

According to the most general embodiment of this invention, the method for carrying out an in vitro cytotoxicity test in the ophthalmic sector comprises first a selecting step wherein a substance to be tested is selected, and one or more concentrations of interest of it (those required for clinical use) are selected.

Before, during or after the selecting step there may be a step of preparing living human cells to be used in the subsequent steps of the method, a preparing step to which this description returns below.

The actual start of the method according to this invention consists of a treatment step, which is carried out in vitro and in a liquid phase, wherein the substance, in the concentration of interest, is put in contact with the living human cells, and is kept in contact with the human cells, in predetermined incubating conditions for a predetermined incubating time interval.

According to this invention, as already indicated, the human cells used in the treatment step are constituted of one or more first pieces of tissue obtained from a tissue constituted of an optical part of a human retina. Moreover, in particular, it is an optical part of a human retina which has been obtained from a human eyeball which has been explanted from a deceased subject.

Moreover, it is appropriate that the treatment step starts within seventy-two hours of the death of the subject, preferably within forty-eight hours thereof. In that period of time the optical part of the retina must be detached from the rest of the eyeball, and the one or more first pieces of tissue must be obtained from it (preferably in the ways described below).

It should be noticed that according to this invention, each piece of the optical part of the retina used in a step of the method must be understood to be a piece covering the entire thickness of the retina and therefore which includes all of the layers of which the retina is constituted.

In the preferred embodiments, each piece of tissue has a maximum width of between 1 mm and 10 mm, preferably between 2 mm and 4 mm. The tests carried out until now have highlighted how disk-shaped pieces of tissue with a 3 mm diameter give the best results.

The predetermined incubating conditions are, in general, conditions suitable for promoting cell survival and/or proliferation. The predetermined incubating conditions provide for the immersion of each piece of tissue in a liquid culture medium. It should be noticed that in the whole context of this description and of the appended claims, any generic reference to a generic "piece of tissue", must be understood to relate to each of the different pieces of tissue usable in one of the steps of the method (as will be seen below indeed there may be first pieces of tissue, second pieces of tissue, third pieces of tissue and fourth pieces of tissue present). Furthermore, any generic reference to a "substance", must be understood to relate to each of the substances usable in one of the steps of the method (as will be seen below indeed it may be the substance to be tested, a cytotoxic substance or a non-cytotoxic substance).

The predetermined incubating conditions also provide for keeping the temperature in a predetermined range, usually in a range suitable for promoting cell survival and/or proliferation.

In the preferred embodiment, the predetermined incubating conditions provide for incubation in an incubator with 5% ± 1% of CO₂, and the use of a liquid culture medium constituted at least of DMEM/F-12 (Dulbecco's Modified Eagle Medium/Nutrient Mixture F-12) with the addition of:
- inactivated Bovine Calf Serum (BCS) in a quantity equal to 10% volume/final volume; and
- Penicillin - Streptomycin Solution in a quantity equal to 1% volume/final volume so as to have a final concentration of 100 IU/ml of penicillin and of 100 µg/ml of streptomycin.

Moreover, the temperature must remain in the range 37°C ± 1°C and the humidity greater than 90%.

Moreover, according to this invention, the predetermined incubating time interval is advantageously not less than twenty-four hours.

Once the treatment step has ended, the method according to this invention provides for an evaluating step wherein the viability of the human cells which have been subjected to the treatment step is evaluated.

In the preferred embodiments, the evaluating step is carried out with a colorimetric technique which provides for the use of a dye capable of selectively staining either only the living cells or only the dead cells. Advantageously, the colorimetric technique uses as a dye MTT (also known as: tetrazolium salt; 2H-Tetrazolium, 2-(4,5-dimethyl-2-thiazolyl)-3,5-diphenyl-, bromide; CAS NUMBER 298-93-1), Neutral Red, XTT (2,3-bis(2-methoxy-4-nitro-5-sulfophenyl)-5-[(phenylamino)carbonyl] -2H-tetrazolium hydroxide), or bicinchoninic acid (BCA).

Moreover, in preferred embodiments, the viability of the cells is evaluated by extracting the dye from the cells, creating a solution with it and measuring an absorbance (optical density) value of that solution.

In more detail, in the currently preferred embodiment, the evaluating step for evaluating the viability of the cells of each piece of tissue, in turn comprises the following operating steps, which are carried out in the order indicated:
- a removing step wherein the piece of tissue is removed from the predetermined incubating conditions;
- a washing step wherein the piece of tissue is washed with a Ca++ Mg++ phosphate buffer sterile solution;
- an immersing step wherein the piece of tissue is immersed in a sterile immersing solution containing MTT, and is left immersed in predetermined immersing conditions and for a predetermined immersing time interval; advantageously, the predetermined immersing conditions provide for immersion in a solution of MTT in DMEM/F-12 without supplements having a concentration of 1 mg/ml, at a temperature in the range 37°C ± 1°C, with a humidity greater than 90% and in an incubator with 5% ± 1% of CO₂; in contrast, the predetermined immersing time interval is equal to at least three hours. It should be noticed that the MTT is a salt soluble in water, for forming a yellow-coloured solution. It also has the property that mitochondrial succinate dehydrogenase and cytoplasmic dehydrogenase metabolise it into formazan salt, a blue-coloured crystalline product, which in contrast is insoluble in water. Using MTT in the context of this invention, only the cells which survived the treatment step are capable of metabolising it, so that the quantity of formazan salt which can be found at the end of the immersing step is proportionate to the number of living cells present in the piece of tissue being examined, and may be used to quantify the viability of the piece of tissue.
- a washing step wherein the piece of tissue is extracted from the sterile immersing solution, and is washed with a Ca++ Mg++ phosphate buffer sterile solution in order to eliminate any dye residues;
- an extracting step wherein each piece of tissue is immersed in isopropanol, and is subjected to slow agitation (preferably with a plate agitator) in predetermined extracting conditions and for a predetermined extracting time interval, for extracting the dye contained in it and obtaining a blue solution of the dye (the formazan salt) in isopropanol. Advantageously the predetermined extracting conditions provide for it to be carried out at ambient temperature (which means any temperature between 20°C and 30°C) and the predetermined extracting time interval is equal to at least two hours. At the end of the two hours, the formazan salt formed in the living cells is transferred to the isopropanol with which it has formed a blue-coloured solution; the intensity of the colour is proportionate to the number of living cells.
- finally a spectrophotometric reading step during which an absorbance value of the solution of dye in isopropanol is measured. Advantageously the spectrophotometric reading is carried out at 570 nm, on a sample of the isopropanol solution without the piece of tissue, and is carried out after that sample has been left being agitated for at least ten minutes.

In order to evaluate whether the results of the evaluating step may be considered reliable, the method according to this invention also provides for carrying out two reliability control tests. Advantageously the two control tests may be carried out as suggested by ISO-10993-5, that is to say by direct contact.

The first control test provides for a positive control step, wherein one or more second pieces of tissue, obtained from the same tissue from which the first pieces of tissue have been obtained, are placed in contact with a cytotoxic substance in a relative concentration of interest (that is to say, such that it guarantees a known cytotoxic effect on the retinal cells) and are kept in contact with the cytotoxic substance, in predetermined positive control conditions, and for a predetermined positive control time interval. The positive control step is also carried out in vitro and in a liquid phase, and is performed independently for each second piece of tissue. The aim of this positive control step is to demonstrate the capacity of the retinal tissue cells used to supply a suitable response if subjected to a cytotoxic substance.

In contrast, the second control test provides for a negative control step, wherein one or more third pieces of tissue, obtained from the same tissue from which the first pieces of tissue have been obtained, are placed in contact with a non-cytotoxic substance in a relative concentration of interest (that is to say, such that it guarantees a known non-cytotoxic effect on the retinal cells, that is to say, the substance can be considered safe for the body) and are kept in contact with the non-cytotoxic substance, in predetermined negative control conditions, and for a predetermined negative control time interval. The negative control step is also carried out in vitro and in a liquid phase, and is performed independently for each third piece of tissue. The aim of this positive control step is again to demonstrate the capacity of the retinal tissue cells used to supply a suitable response (none) if subjected to a non-cytotoxic substance.

In the preferred embodiments, both the positive control step, and the negative control step are carried out by direct contact between the control substance (cytotoxic or non-cytotoxic), in the concentration of interest, and, respectively, the second and the third pieces of tissue. For example, that may be achieved by covering each piece of tissue with a predetermined quantity of the control substance (for example constituted of a perfluorinated liquid) and then covering the control substance with a different substance having lower density (for example complete culture medium). Indeed, thanks to the different densities of the substances, the control substances remain in contact with the piece of tissue and do not move upwards. The duration of the test may be at least 24h at +37°C±1°C in an incubator with 5%±1°C CO2, and humidity greater than 90%).

Once the two control tests have been carried out, the method therefore provides for the evaluating step to also be carried out for each second piece of tissue and each third piece of tissue, so as to verify that the related results in terms of viability, are those expected.

After the evaluating step, the method provides for carrying out a determining step wherein, based on the previously evaluated viability for the first pieces of tissue, the cytotoxicity or non-cytotoxicity of the substance to be tested is determined.

In general, the determining step is carried out by considering the substance to be tested to be cytotoxic if, based on the results of the evaluating step, it can be determined that, during the treatment step, the substance to be tested has been responsible for a reduction in the viability of the cells of the one or more first pieces of tissue, which is above a predetermined threshold. Preferably, the predetermined threshold is equal to 30%. Moreover, the reduction in viability is advantageously evaluated, not relative to the viability of the same piece of tissue at the start of the method, but relative to the viability of other pieces of tissue which have been subjected to a similar treatment step, but in the absence of the substance to be tested.

For that purpose, in its preferred embodiment, the method also comprises a comparison result defining step, wherein one or more fourth pieces of tissue, obtained from the same tissue from which the first pieces of tissue have been obtained, are kept immersed in only the culture medium, in the predetermined incubating conditions and for the predetermined incubating time interval. The comparison result defining step is also carried out in vitro and is performed independently for each fourth piece of tissue.

Then, the evaluating step is also carried out for each of the one or more fourth pieces of tissue which have been subjected to the comparison result defining step.

Finally, the determining step for determining the cytotoxicity or non-cytotoxicity of the substance to be tested, is carried out as a comparison between the evaluated viability of the cells of the one or more first pieces of tissue, and the evaluated viability of the cells of the one or more fourth pieces of tissue.

Advantageously, that step of determining the cytotoxicity or non-cytotoxicity of the substance to be tested, is carried out by calculating a ratio of a first value, representative of the viability of the cells of the one or more first pieces of tissue, to a second value, representative of the viability of the cells of the one or more fourth pieces of tissue.

Furthermore, the substance to be tested is considered cytotoxic when that ratio is less than a predetermined limit value, preferably equal to 0.7 (or 70% if expressed as a percentage).

In any case, in order to be able to consider the results obtained reliable, it is appropriate for the absorbance value measured for the fourth pieces of tissue to be equal to or greater than 0.3.

In order to avoid any interference effects caused by the ways in which the various steps of the process are carried out (for example the tissue, or the container used), in the preferred embodiment the viability measurements are corrected relative to those possible interference effects.

Consequently, there may be a further, reference defining step, wherein at least one sample of only the culture medium is kept in the predetermined incubating conditions, for the predetermined incubating time interval (preferably using the same containers used for the pieces of tissue). At the end of the predetermined incubating time interval, that sample of only the culture medium is used for determining the viability of the cells of the one or more first pieces of tissue and of the cells of the one or more fourth pieces of tissue, more specifically for correcting the viability evaluations carried out for the first and for the fourth pieces of tissue.

In particular, in the case of carrying out a spectrophotometric reading step for determining the viability of the cells, an absorbance value is also measured for the sample of only the culture medium. That value is then used in the calculation of the ratio provided in the step for determining the cytotoxicity or non-cytotoxicity of the substance to be tested. In particular it is used both for calculating the first value of the ratio (the numerator), which is calculated as the difference between the absorbance value measured for the one or more first pieces of tissue, and that measured for the sample of culture medium, and for similarly calculating the second value of the ratio (the denominator), which is also calculated as the difference between the absorbance value measured for the one or more fourth pieces of tissue, and that measured for the sample of culture medium.

Finally, it should be remembered that if carrying out the control steps described above is provided for, in the determining step, the cytotoxicity or non-cytotoxicity of the substance to be tested is evaluated by also taking into account the results of the evaluating step carried out on the one or more second pieces of tissue and on the one or more third pieces of tissue. In particular, the determining step is only carried out if the results of the positive and negative control steps have been consistent with expectations (that is to say, if the viability of the second pieces of tissue - positive control - is less than 70% and if that of the third pieces of tissue - negative control - is greater than or equal to 70%).

Finally, it should be noticed that where the treatment step, the control steps and/or the comparison result defining step are carried out in parallel, and independently, on a plurality of separate pieces of tissue, in the respective evaluating steps, the overall viability of the cells is evaluated by taking into account the results of the individual measurements, for example as an average value of the viability evaluated for each piece of tissue.

Finally, depending on the requirements of the tester, it must be emphasised that, according to this invention, the decision about the cytotoxicity or non-cytotoxicity of a specific substance to be tested may be made either by implementing the method for carrying out an in vitro cytotoxicity test in the ophthalmic sector once, or by carrying it out multiple times each time using a different human retina, without prejudice to the fact that all of the pieces of tissue used for each execution must have been obtained from the optical part of a single retina. Therefore, the decision may be made to consider the substance to be tested cytotoxic if this is the case at least one of the times the method is carried out.

Although what is described above may even be carried out starting with pieces of tissue previously prepared by others, as already indicated, the embodiment of this invention also provides for a step of preparing pieces of tissue to be used (that is to say, human cells).

First, that step requires the explanted eyeball to be transferred from the explanting location to the laboratory assigned for its processing, either whole or, if necessary, without the cornea (if the latter may be intended to be used as a transplant). Preferably, the eyeballs must be transferred within forty-eight hours of death (within twenty-four hours is better), they must be transported at 4°C, in sterile hermetically sealed containers (advantageously 30 ml size), and they must be kept immersed in a DMEM/F-12 culture medium for transport at +4°C.

Once the processing laboratory has been reached, the retina must be extracted within twenty-four hours at the most, that is to say, within seventy-two hours of death, but preferably within forty-eight hours of death.

The retina is extracted from the eyeball preferably whole, and in sterile conditions, advantageously under ISO-5 Biohazard laminar flow hoods.

The extraction steps are as follows:
1. Washing of the whole eyeball with a culture medium, for example composed of DMEM/F-12 medium, supplemented, in a volume/final volume, with 10% of Fetal Calf Serum (FCS) and 1% of a Penicillin - streptomycin solution (so as to have a final concentration of 100 IU/ml of penicillin and of 100 µg/ml of streptomycin).
2. Extraction of the crystalline lens, for example using blunt-ended scissors and fine anatomical forceps.
3. Dissection of the sclera, for example using blunt-ended scissors and fine forceps, so as to separate the choroid with the retina and the whole vitreous body from the scleral tissue.
4. Separation of the whole retina from the choroid and from the vitreous body, for example using fine anatomical forceps; preferably first the whole choroid separates from the retina and vitreous body, then the whole vitreous body detaches from the retina (or from only its optical part) by washing with the culture medium.
5. Preparation of the pieces of retinal tissue for the cytotoxicity test: the retinal tissue is stretched out (for example in a sterile Petri dish) and culture medium is added, for keeping the retina hydrated. Then full-thickness punching/coring is carried out at the optical part of the retina, using biopsy punches, with a diameter range of from 2 mm to 10 mm, preferably with a diameter of 3 mm. In particular, in this step efforts are made to obtain the maximum possible number of pieces of tissue.

Finally, all of the pieces of tissue which have been obtained can be inserted, one per well, in the wells of a single plate, for example using a Gilson positive displacement pipette (since it is very gentle on the retinal tissue), to avoid risking damaging them.

In this step it may be useful to already divide the pieces of tissue into groups of first pieces of tissue, second pieces of tissue, third pieces of tissue and fourth pieces of tissue in view of carrying out the subsequent steps of the method.

The tests carried out up to now by the Applicant have highlighted that, from a single optical part of a retina, it is usually possible to obtain at least twenty-four circular pieces of tissue with 3 mm diameter.

It was also ascertained that such pieces of tissue may advantageously be divided as follows:
- between 8 and 16 first pieces of tissue;
- between 8 and 16 second pieces of tissue;
- between 8 and 16 third pieces of tissue;
- between 8 and 16 fourth pieces of tissue.

In any case, it is preferable that each type comprises at least six pieces of tissue.

This invention brings important advantages.

In particular, thanks to this invention it has been possible to provide a method for carrying out an in vitro cytotoxicity test in the ophthalmic sector, which guarantees much greater reliability than the prior art methods, and in particular than the prior art method which involves the use of a cell culture of retinal pigmented epithelial cells.

In order to highlight that advantage of this invention, that is to say, the greater sensitivity of the new method compared with the prior art method based on ARPE-19 cells, both direct contact cytotoxicity tests with perfluorocarbons, and cytotoxicity tests with two commercial 1000 cts silicone oils (hereinafter generically called SILICONE OIL 1 and SILICONE OIL 2) have been carried out.

The compounds used as positive controls (indicators of cell death) were:
1) POSITIVE CONTROL 1: 1H-perfluorooctane (CAS 335-65-9) at 12.5% diluted in ultrapure perfluorooctane;
2) POSITIVE CONTROL 2: Perfluorooctanoic acid (CAS 335-67-1) at 0.01% in ultrapure perfluorooctane.

In contrast, for the NEGATIVE CONTROL a 100% pure perfluorooctane (HPF8, produced by Alchimia srl - lot P181 00018) was used.

Advantageously, the direct contact cytotoxicity tests were carried out by covering each piece of tissue with a quantity of between 50 and 100 microlitres of the substance and then covering the substance with around 200 microlitres of complete culture medium. Thanks to the different densities of the substances and of the culture medium, the substances remain in contact with the piece of tissue and do not move upwards. The duration of the test was 24h at +37°C±1°C in an incubator with 5%±1°C CO2, and humidity greater than 90%).

The final results of the test, in terms of residual cell viability compared with untreated samples (evaluated with MTT-TEST 570nm absorbance measurements as described above), are shown in the table below, where the data which highlight cytotoxicity are underlined:

| | **% RESIDUAL CELL VIABILITY** | | | | |
|---|---|---|---|---|---|
| | **NEGATIVE CONTROL 1** | **POSITIVE CONTROL 1** | **POSITIVE CONTROL 2** | **1000 cts SILICONE OIL 1** | **1000 cts SILICONE OIL 2** |
| **RETINA 1 (PUNCH 3MM)** | 95% (N=16) | 29% (N=16) | 18.5% (N=16) | 89% (N=11) | 69% (N=15) |
| **RETINA 2 (PUNCH3MM)** | 96% (N=16) | 30% (N=16) | 16.1% (N=16) | 90% (N=12) | 60% (N=12) |
| **ARPE-19 CELLS** | 95% (N=24) | 69.7% (N=24) | 57.4% (N=16) | 92% (N=24) | 98% (N=24) |

| | | | | | |
|---|---|---|---|---|---|
| where N indicates the number of tests carried out (which corresponds to the number of pieces of tissue used). | | | | | |

The data highlight the match between the results of the two methods as regards the negative control, whilst the method according to this invention is more precise than the prior art method which uses ARPE-19 cells as regards the two positive controls for which the test carried out using ARPE-19 highlighted a much less pronounced cytotoxicity.

Furthermore, the method according to this invention highlighted a cytotoxicity (on both of the retinas tested) even with reference to one of the two commercial silicone oils, a cytotoxicity that in contrast was not highlighted by the test carried out using ARPE-19 which therefore proved much less sensitive.

Finally, it should be noticed that this invention is relatively easy to produce and that even the cost linked to implementing the invention is not very high.

## Claims

1. Method for carrying out an in vitro cytotoxicity test in the ophthalmic sector, comprising the following operating steps:
a selecting step wherein a substance to be tested and a concentration of interest of it are selected;
a treatment step, carried out in vitro and in a liquid phase, wherein the substance to be tested, in the concentration of interest, is put in contact with living human cells, and is kept in contact with the human cells, in predetermined incubating conditions for a predetermined incubating time interval, wherein said predetermined incubating conditions provide for the immersion of each piece of tissue in a liquid culture medium containing the respective substance in the concentration provided for, and keeping the temperature in a predetermined range;
an evaluating step wherein, once the predetermined incubating time interval has elapsed, the viability of the human cells subjected to the treatment step is evaluated; and
a determining step wherein, based on the previously evaluated viability, the cytotoxicity or non-cytotoxicity of the substance to be tested is determined; wherein:
for carrying out the method use is made of human cells constituted of one or more first pieces of tissue, wherein the tissue is an optical part of a human retina;
the one or more first pieces of tissue are obtained from a human eyeball and each comprise the full thickness of the optical part of the retina;
the human eyeball is an eyeball explanted from a deceased subject; and
the treatment step starts within seventy-two hours of the death of the deceased subject.

2. The method according to claim 1, wherein in the determining step the substance to be tested is considered cytotoxic if, based on the results of the evaluating step, it is determined that during the treatment step it has been responsible for a reduction in the viability of the human cells which is above a predetermined threshold, preferably above a predetermined threshold of 30%.

3. The method according to claim 1 or 2 also comprising the following steps:
a positive control step, wherein one or more second pieces of tissue obtained from the same tissue, in vitro and each independently, are placed in contact with a cytotoxic substance, present in a cytotoxic concentration, and are kept in contact with the cytotoxic substance, in predetermined positive control conditions, and for a predetermined positive control time interval;
a negative control step, wherein one or more third pieces of tissue obtained from the same tissue, in vitro and each independently, are placed in contact with a non-cytotoxic substance, present in a non-cytotoxic concentration, and are kept in contact with the non-cytotoxic substance, in predetermined negative control conditions, and for a predetermined negative control time interval;
wherein the evaluating step is also carried out for each second piece of tissue and each third piece of tissue, and wherein in the determining step, the cytotoxicity or non-cytotoxicity of the substance to be tested is evaluated by also taking into account the results of the evaluating step carried out on the one or more second pieces of tissue and on the one or more third pieces of tissue.

4. The method according to claim 1, 2 or 3 also comprising a comparison result defining step, wherein one or more fourth pieces of tissue obtained from the same tissue, in vitro and each independently, are kept immersed in only the culture medium, in the predetermined incubating conditions and for the predetermined incubating time interval;
wherein the evaluating step is also carried out for each of the one or more fourth pieces of tissue;
and wherein the determining step for determining the cytotoxicity or non-cytotoxicity of the substance to be tested is carried out as a comparison between the evaluated viability of the cells of the one or more first pieces of tissue and the evaluated viability of the cells of the one or more fourth pieces of tissue.

5. The method according to claim 4 wherein the determining step for determining the cytotoxicity or non-cytotoxicity of the substance to be tested, is carried out by calculating a ratio of a first value, representative of the viability of the cells of the one or more first pieces of tissue, to a second value, representative of the viability of the cells of the one or more fourth pieces of tissue, and wherein the substance to be tested is considered cytotoxic when that ratio is less than a predetermined limit value, preferably equal to 0.7.

6. The method according to either of claims 4 or 5 also comprising a reference defining step, wherein at least one sample of only the culture medium is kept in the predetermined incubating conditions for the predetermined incubating time interval, and wherein at the end of the predetermined incubating time interval the at least one sample is used for determining the viability of the cells of the one or more first pieces of tissue and of the cells of the one or more fourth pieces of tissue.

7. The method according to claim 6 wherein each evaluating step for evaluating the viability provides for the use of a colorimetric technique, and wherein the viability of the cells is measured by measuring an absorbance value of a solution obtained by extracting dye from the piece of tissue to be evaluated, wherein an absorbance value is also measured for said at least one sample of culture medium, and wherein in the calculation of the ratio provided for in the determining step for determining the cytotoxicity or non-cytotoxicity of the substance to be tested, the first value is calculated as the difference between the absorbance value measured for the one or more first pieces of tissue and that measured for the sample of culture medium, and the second value is calculated as the difference between the absorbance value measured for the one or more fourth pieces of tissue and that measured for the sample of culture medium.

8. The method according to any of claims 1 to 6 wherein each evaluating step for evaluating the viability provides for the use of a colorimetric technique.

9. The method according to claim 7 or 8 wherein the colorimetric technique uses MTT as the dye.

10. The method according to claim 9 wherein, at the end of the predetermined incubating time interval, the evaluating step for evaluating the viability of the cells of each piece of tissue, in turn comprises the following operating steps in the order indicated:
a removing step wherein the piece of tissue is removed from the predetermined incubating conditions;
a washing step wherein the piece of tissue is washed with a Ca⁺⁺ Mg⁺⁺ phosphate buffer sterile solution;
an immersing step wherein the piece of tissue is immersed in a sterile immersing solution containing MTT, and is left immersed in predetermined immersing conditions and for a predetermined immersing time interval;
a washing step wherein the piece of tissue is extracted from the sterile immersing solution and is washed with a Ca⁺⁺ Mg⁺⁺ phosphate buffer sterile solution in order to eliminate any dye residues;
an extracting step wherein each piece of tissue is immersed in isopropanol, and is subjected to slow agitation in predetermined extracting conditions and for a predetermined extracting time interval, for extracting the dye contained in it and obtaining a solution of dye in isopropanol;
a spectrophotometric reading step during which an absorbance value of the solution of dye in isopropanol is measured.

11. The method according to any of claims 1 to 10 wherein, in the evaluating step, the viability of the human cells is evaluated as the average of the viability evaluated for each piece of tissue.

12. The method according to any of claims 1 to 11 wherein each piece of tissue has a maximum width of between 1 mm and 10 mm, preferably between 2 mm and 4 mm.

13. The method according to any of claims 1 to 12, also comprising, before the selecting step, a step of preparing pieces of tissue which in turn provides for the following steps:
explanting of an eyeball from a deceased subject;
preserving of the explanted eyeball in a DMEM/F-12 culture medium at a nominal temperature of +4°C;
extracting of the optical part of the retina from the eyeball within seventy-two hours of the death, preferably within forty-eight hours of the death;
obtaining pieces of retinal tissue from the optical part of the retina by full-thickness punching/coring, with a diameter range of from 2 mm to 10 mm, preferably with a diameter range of from 2.5 mm to 3.5 mm.

14. A method for evaluating the cytotoxicity of a substance, wherein a method according to any of claims 1 to 13 is carried out multiple times for the same substance to be tested, each time using a single optical part of a different human retina, and wherein the substance to be tested is considered cytotoxic if that is the result of the determining step of at least one of the times the method according to any of claims 1 to 13 is carried out.

## Patentansprüche

1. Verfahren zur Durchführung eines In-Vitro-Zytotoxizitätstests im Bereich der Augenheilkunde, folgende Arbeitsschritte beinhaltend:
einen Auswahlschritt, bei dem eine zu testende Substanz und eine Konzentration von Interesse davon ausgewählt werden;
einen Behandlungsschritt, der in vitro und in flüssiger Phase durchgeführt wird, bei dem die zu testende Substanz in der Konzentration von Interesse mit lebenden humanen Zellen in Kontakt gebracht wird und unter vorher festgelegten Inkubationsbedingungen über einen vorher festgelegten Inkubationszeitraum mit den humanen Zellen in Kontakt gehalten wird, wobei besagte vorher festgelegte Inkubationsbedingungen die Immersion jedes Gewebeteils in ein flüssiges Kulturmedium, das die jeweilige Substanz in der vorgesehenen Konzentration enthält, und die Erhaltung der Temperatur innerhalb eines vorher festgelegten Bereichs vorsehen;
einen Auswertungsschritt, bei dem, sobald der vorher festgelegte Inkubationszeitraum abgelaufen ist, die Viabilität der humanen Zellen, die der Behandlung unterzogen wurden, bewertet wird;
und
einen Bestimmungsschritt, auf der vorher ausgewerteten Viabilität basierend, bei dem die Zytotoxizität oder Nicht-Zytotoxizität der zu testenden Substanz bestimmt wird, wobei: um das Verfahren durchzuführen, Gebrauch von humanen Zellen von einem oder mehreren ersten Gewebeteilen gemacht wird, wobei das Gewebe ein optischer Teil einer humanen Netzhaut ist;
das eine oder die mehreren ersten Gewebeteile aus einem humanen Augapfel gewonnen wurden und jedes davon die vollständige Dicke des optischen Teils der Netzhaut umfasst;
der humane Augapfel ein Augapfel ist, der von einer verstorbenen Person explantiert wurde; und der Behandlungsschritt innerhalb von zweiundsiebzig Stunden nach dem Tod der verstorbenen Person beginnt.

2. Das Verfahren nach dem Patentanspruch 1, wobei die zu testende Substanz beim Bestimmungsschritt als zytotoxisch angesehen wird, wenn - aufgrund der Ergebnisse des Auswertungsschritts - festgestellt wird, dass sie während des Behandlungsschritts für eine Senkung der Viabilität der humanen Zellen verantwortlich war, die oberhalb eines vorher festgelegten Schwellenwerts liegt, vorzugsweise oberhalb eines vorher festgelegten Schwellenwerts von 30 %.

3. Das Verfahren nach dem Patentanspruch 1 oder 2, auch folgende Schritte beinhaltend: einen Schritt der Positivkontrolle, bei dem ein oder mehrere zweite Gewebeteile, die aus demselben Gewebe gewonnen wurden, in vitro und jedes unabhängig von den anderen in Kontakt mit einer zytotoxischen Substanz, die in einer zytotoxischen Konzentration vorliegt, gebracht werden und unter vorher festgelegten Bedingungen für die Positivkontrolle, und über einen vorher festgelegten Zeitraum für die Positivkontrolle mit der zytotoxischen Substanz in Kontakt gehalten werden; einen Schritt der Negativkontrolle, bei dem ein oder mehrere dritte Gewebeteile, die aus demselben Gewebe gewonnen wurden, in vitro und jedes unabhängig von den anderen in Kontakt mit einer nicht-zytotoxischen Substanz, die in einer nicht-zytotoxischen Konzentration vorliegt, gebracht werden und unter vorher festgelegten Bedingungen für die Negativkontrolle, und über einen vorher festgelegten Zeitraum für die Negativkontrolle mit der nicht-zytotoxischen Substanz in Kontakt gehalten werden;
wobei der Auswertungsschritt auch für jedes zweite Gewebeteil und für jedes dritte Gewebeteil durchgeführt wird, und wobei beim Bestimmungsschritt die Zytotoxizität oder Nicht-Zytotoxizität der zu testenden Substanz bewertet wird, indem auch die Ergebnisse des Auswertungsschritts, der an dem einen oder den mehreren zweiten Gewebeteilen und an dem einen oder den mehreren dritten Gewebeteilen durchgeführt wurde, berücksichtigt werden.

4. Das Verfahren nach dem Patentanspruch 1, 2 oder 3, auch einen Schritt zur Bestimmung des Vergleichsergebnisses beinhaltend, bei dem ein oder mehrere vierte Gewebeteile, die aus demselben Gewebe gewonnen wurden, in vitro und jedes unabhängig von den anderen, unter den vorher festgelegten Inkubationsbedingungen und über den vorher festgelegten Inkubationszeitraum nur in dem Kulturmedium eingetaucht bleiben;
wobei der Auswertungsschritt auch für jedes der ein oder mehreren vierten Gewebeteile durchgeführt wird;
und wobei der Bestimmungsschritt zur Bestimmung der Zytotoxizität oder Nicht-Zytotoxizität der zu testenden Substanz als Vergleich zwischen der ausgewerteten Viabilität der Zellen des einen oder der mehreren ersten Gewebeteile und der ausgewerteten Viabilität des einen oder der mehreren vierten Gewebeteile durchgeführt wird.

5. Das Verfahren nach dem Patentanspruch 4, wobei der Bestimmungsschritt zur Bestimmung der Zytotoxizität oder Nicht-Zytotoxizität der zu testenden Substanz durch Berechnung eines Verhältnisses eines ersten Wertes, der die Viabilität der Zellen des einen oder der mehreren ersten Gewebeteile repräsentiert, zu einem zweiten Wert, der die Viabilität der Zellen des einen oder der mehreren vierten Gewebeteile repräsentiert, durchgeführt wird, und wobei die zu testende Substanz als zytotoxisch angesehen wird, wenn dieses Verhältnis unterhalb eines vorher festgelegten Grenzwerts liegt, vorzugsweise gleich 0,7.

6. Das Verfahren nach beiden Patentansprüchen 4 oder 5, auch einen Schritt zur Referenzbestimmung beinhaltend, bei dem mindestens eine Probe nur des Kulturmediums über den vorher festgelegten Inkubationszeitraum unter den vorher festgelegten Inkubationsbedingungen aufbewahrt wird, und bei dem am Ende des vorher festgelegten Inkubationszeitraums die mindestens eine Probe verwendet wird, um die Viabilität der Zellen des einen oder der mehreren ersten Gewebeteile und der Zellen des einen oder der mehreren vierten Gewebeteils zu bestimmen.

7. Das Verfahren nach dem Patentanspruch 6, wobei jeder Auswertungsschritt zur Bewertung der Viabilität die Anwendung einer kolorimetrischen Methode vorsieht, und wobei die Zellviabilität durch Messung eines Absorptionswerts einer Lösung gemessen wird, die durch Extraktion von Farbstoff aus dem auszuwertenden Gewebeteil erhalten wird, wobei ein Absorptionswert auch für besagte mindestens eine Probe des Kulturmediums gemessen wird, und wobei für die Berechnung des Verhältnisses, das im Bestimmungsschritt zur Bestimmung der Zytotoxizität oder der Nicht-Zytotoxizität der zu testenden Substanz vorgesehen ist, der erste Wert als Unterschied zwischen dem Absorptionswert, der für das eine oder die mehreren ersten Gewebeteile gemessen wurde, und demjenigen, der für die Probe des Kulturmediums gemessen wurde, berechnet wird, und der zweite Wert als Unterschied zwischen dem an dem einen oder den mehreren vierten Gewebeteilen gemessenen Absorptionswert und demjenigen, der für die Probe des Kulturmediums gemessen wurde, berechnet wird.

8. Das Verfahren nach jedem der Patentansprüche 1 bis 6, wobei jeder Auswertungsschritt zur Bewertung der Viabilität die Anwendung einer kolorimetrischen Methode vorsieht.

9. Das Verfahren nach dem Patentanspruch 7 oder 8, wobei die kolorimetrische Methode MTT als Farbstoff verwendet

10. Das Verfahren nach dem Patentanspruch 9, wobei am Ende des vorher festgelegten Inkubationszeitraums der Auswertungsschritt zur Bewertung der Viabilität der Zellen jedes Gewebeteils seinerseits folgende Arbeitsschritte, in der angegebenen Reihenfolge, beinhaltet:
einen Entnahmeschritt, bei dem das Gewebeteil aus den vorher festgelegten Inkubationsbedingungen entnommen wird;
einen Spülschritt, bei dem das Gewebeteil mit einer sterilen Ca++-Mg++-Phosphat-Pufferlösung gespült wird;
einen Eintauchschritt, bei dem das Gewebeteil in eine sterile Immersionslösung, die MTT enthält, eingetaucht wird und unter vorher festgelegten Immersionsbedingungen und über einen vorher festgelegten Immersionszeitraum darin eingetaucht bleibt;
einen Spülschritt, bei dem das Gewebeteil aus der sterilen Immersionslösung entnommen wird und mit einer sterilen Ca++-Mg++-Phosphat-Pufferlösung gespült wird, um jegliche Farbstoffreste zu eliminieren;
einen Extraktionsschritt, bei dem jedes Gewebeteil in Isopropanol eingetaucht wird und unter vorher festgelegten Extraktionsbedingungen und über einen vorher festgelegten Extraktionszeitraum einer langsamer Bewegung unterzogen wird, um den Farbstoff, der darin enthalten ist, zu extrahieren und eine Farbstofflösung in Isopropanol zu gewinnen;
einen Schritt der spektralphotometrischen Erfassung, bei dem ein Absorptionswert der Farbstofflösung in Isopropanol gemessen wird.

11. Das Verfahren nach jedem der Patentansprüche 1 bis 10, wobei bei dem Auswertungsschritt die Viabilität der humanen Zellen als Durchschnitt der Viabilität, die für jedes einzelne Gewebeteil ausgewertet wurde, ausgewertet wird.

12. Das Verfahren nach jedem der Patentansprüche 1 bis 11, wobei jedes einzelne Gewebeteil eine maximale Breite zwischen 1 mm und 10 mm hat, vorzugsweise zwischen 2 mm und 4 mm.

13. Das Verfahren nach jedem der Patentansprüche 1 bis 12, vor dem Auswahlschritt auch einen Schritt des Vorbereitens von Gewebeteilen beinhaltend, der seinerseits folgende Schritte vorsieht:
Explantation eines Augapfels von einer verstorbenen Person, Konservierung des entnommenen Augapfels in einem DMEM/F-12-Kulturmedium bei einer Solltemperatur von +4° C;
Entnahme des optischen Teils der Netzhaut aus dem Augapfel innerhalb von zweiundsiebzig Stunden nach dem Tod, vorzugsweise innerhalb von achtundvierzig Stunden nach dem Tod;
Gewinnung von Stücken des Netzhautgewebes aus dem optischen Teil der Netzhaut durch Ausstanzen/Coring über die gesamte Dicke mit einem Durchmesserbereich von 2 mm bis 10 mm, vorzugsweise innerhalb eines Durchmesserbereichs zwischen 2,5 mm und 3,5 mm.

14. Ein Verfahren zur Auswertung der Zytotoxizität einer Substanz, bei dem ein Verfahren nach jedem der Patentansprüche 1 bis 13 mehre Male für dieselbe zu testende Substanz durchgeführt wird, wobei jedes Mal ein einziger optischer Teil einer unterschiedlichen humanen Netzhaut verwendet wird, und wobei die zu testende Substanz als zytotoxisch angesehen wird, wenn dies das Ergebnis des Bestimmungsschritts aus mindestens einem der Male, die das Verfahren nach jedem der Patentansprüche 1 bis 13 durchgeführt wird, ist.

## Revendications

1. Méthode pour l'exécution d'un test de cytotoxicité in vitro dans le domaine ophtalmique, comprenant les étapes opérationnelles suivantes :
une étape de sélection au cours de laquelle une substance à tester et une concentration d'intérêt de celle-ci sont sélectionnées ;
une étape de traitement, effectuée in vitro et dans une phase liquide, au cours de laquelle la substance à tester, dans la concentration d'intérêt, est mise en contact avec des cellules humaines vivantes, et est maintenue en contact avec les cellules humaines, dans des conditions d'incubation prédéfinies pendant un intervalle de temps d'incubation prédéfini, où lesdites conditions d'incubation prédéfinies prévoient l'immersion de chaque morceau de tissu dans un milieu de culture liquide contenant la substance respective dans la concentration prévue, et le maintien de la température dans une plage prédéfinie ;
une étape d'évaluation au cours de laquelle, une fois l'intervalle de temps d'incubation prédéfini écoulé, la viabilité des cellules humaines soumises à l'étape de traitement est évaluée ; et
une étape de détermination au cours de laquelle, sur la base de la viabilité précédemment évaluée, la cytotoxicité ou non-cytotoxicité de la substance à tester est déterminée ;
dans laquelle :
pour l'exécution de la méthode on utilise des cellules humaines constituées d'un ou de plusieurs premiers morceaux de tissu, où le tissu est une partie optique d'une rétine humaine ;
lesdits un ou plusieurs premiers morceaux de tissu sont obtenus à partir d'un globe oculaire humain et comprennent chacun toute l'épaisseur de la partie optique de la rétine ;
le globe oculaire humain est un globe oculaire explanté chez un sujet décédé ; et
l'étape de traitement débute dans les soixante-douze heures à compter de la mort du sujet décédé.

2. La méthode selon la revendication 1, dans laquelle, au cours de l'étape de détermination, la substance à tester est considérée comme étant cytotoxique si, sur la base des résultats de l'étape d'évaluation, il est déterminé qu'au cours de l'étape de traitement elle a été responsable d'une réduction de la viabilité des cellules humaines qui est supérieure à un seuil prédéfini, de préférence supérieure à un seuil prédéfini de 30 %.

3. La méthode selon la revendication 1 ou 2, comprenant également les étapes suivantes :
une étape de contrôle positif, au cours de laquelle un ou plusieurs deuxièmes morceaux de tissu obtenus à partir du même tissu, in vitro et chacun de façon indépendante, sont placés en contact avec une substance cytotoxique, présente dans une concentration cytotoxique, et sont maintenus en contact avec la substance cytotoxique, dans des conditions prédéfinies de contrôle positif, et pendant un intervalle de temps prédéfini de contrôle positif ;
une étape de contrôle négatif, au cours de laquelle un ou plusieurs troisièmes morceaux de tissu obtenus à partir du même tissu, in vitro et chacun de façon indépendante, sont placés en contact avec une substance non cytotoxique, présente dans une concentration non cytotoxique, et sont maintenus en contact avec la substance non cytotoxique, dans des conditions prédéfinies de contrôle négatif, et pendant un intervalle de temps prédéfini de contrôle négatif ;
dans laquelle l'étape d'évaluation est également effectuée pour chaque deuxième morceau de tissu et chaque troisième morceau de tissu, et dans laquelle, au cours de l'étape de détermination, la cytotoxicité ou non-cytotoxicité de la substance à tester est évaluée en tenant compte également des résultats de l'étape d'évaluation effectuée sur lesdits un ou plusieurs deuxièmes morceaux de tissu et sur lesdits un ou plusieurs troisièmes morceaux de tissu.

4. La méthode selon la revendication 1, 2 ou 3, comprenant également une étape de définition d'un résultat de comparaison, au cours de laquelle un ou plusieurs quatrièmes morceaux de tissu obtenus à partir du même tissu, in vitro et chacun de façon indépendante, sont maintenus immergés dans le seul milieu de culture, dans les conditions d'incubation prédéfinies et pendant l'intervalle de temps d'incubation prédéfini ;
dans laquelle l'étape d'évaluation est également effectuée pour chacun desdits un ou plusieurs quatrièmes morceaux de tissu ;
et dans laquelle l'étape de détermination pour déterminer la cytotoxicité ou non-cytotoxicité de la substance à tester est effectuée comme une comparaison entre la viabilité évaluée des cellules desdits un ou plusieurs premiers morceaux de tissu et la viabilité évaluée des cellules desdits un ou plusieurs quatrièmes morceaux de tissu.

5. La méthode selon la revendication 4, dans laquelle l'étape de détermination pour déterminer la cytotoxicité ou non-cytotoxicité de la substance à tester, est effectuée en calculant un rapport entre une première valeur, représentative de la viabilité des cellules desdits un ou plusieurs premiers morceaux de tissu, et une deuxième valeur, représentative de la viabilité des cellules desdits un ou plusieurs quatrièmes morceaux de tissu, et dans laquelle la substance à tester est considérée comme étant cytotoxique lorsque ce rapport est inférieur à une valeur limite prédéfinie, de préférence égale à 0,7.

6. La méthode selon l'une ou l'autre des revendications 4 ou 5, comprenant également une étape de définition d'une référence, au cours de laquelle au moins un échantillon du seul milieu de culture est maintenu dans les conditions d'incubation prédéfinies pendant l'intervalle de temps d'incubation prédéfini, et dans laquelle, à la fin de l'intervalle de temps d'incubation prédéfini, ledit au moins un échantillon est utilisé pour déterminer la viabilité des cellules desdits un ou plusieurs premiers morceaux de tissu et des cellules desdits un ou plusieurs quatrièmes morceaux de tissu.

7. La méthode selon la revendication 6, dans laquelle chaque étape d'évaluation de la viabilité prévoit l'utilisation d'une technique colorimétrique, et dans laquelle la viabilité des cellules est mesurée en mesurant une valeur d'absorbance d'une solution obtenue par extraction d'un colorant du morceau de tissu à évaluer, dans laquelle une valeur d'absorbance est également mesurée pour ledit au moins un échantillon de milieu de culture, et dans laquelle, dans le calcul du rapport prévu lors de l'étape de détermination de la cytotoxicité ou non- cytotoxicité de la substance à tester, la première valeur est calculée comme la différence entre la valeur d'absorbance mesurée pour lesdits un ou plusieurs premiers morceaux de tissu et celle mesurée pour l'échantillon de milieu de culture, et la deuxième valeur est calculée comme la différence entre la valeur d'absorbance mesurée pour lesdits un ou plusieurs quatrièmes morceaux de tissu et celle mesurée pour l'échantillon de milieu de culture.

8. La méthode selon l'une quelconque des revendications de 1 à 6, dans laquelle chaque étape d'évaluation de la viabilité prévoit l'utilisation d'une technique colorimétrique.

9. La méthode selon la revendication 7 ou 8, dans laquelle la technique colorimétrique utilise le MTT comme colorant.

10. La méthode selon la revendication 9, dans laquelle, à la fin de l'intervalle de temps d'incubation prédéfini, l'étape d'évaluation de la viabilité des cellules de chaque morceau de tissu, comprend à son tour les étapes opérationnelles suivantes dans l'ordre indiqué :
une étape de retrait au cours de laquelle le morceau de tissu est retiré des conditions d'incubation prédéfinies ;
une étape de lavage au cours de laquelle le morceau de tissu est lavé avec une solution stérile de tampon phosphate Ca⁺⁺ Mg⁺⁺ ;
une étape d'immersion au cours de laquelle le morceau de tissu est immergé dans une solution d'immersion stérile contenant du MTT, et est laissé immergé dans des conditions d'immersion prédéfinies et pendant un intervalle de temps d'immersion prédéfini ;
une étape de lavage au cours de laquelle le morceau de tissu est extrait de la solution d'immersion stérile et est lavé avec une solution stérile de tampon phosphate Ca⁺⁺ Mg⁺⁺ afin d'éliminer les éventuels résidus de colorant ;
une étape d'extraction au cours de laquelle chaque morceau de tissu est immergé dans de l'isopropanol, et est soumis à agitation lente dans des conditions d'extraction prédéfinies et pendant un intervalle de temps d'extraction prédéfini, afin d'extraire le colorant qui y est contenu et d'obtenir une solution de colorant dans l'isopropanol ;
une étape de lecture spectrophotométrique au cours de laquelle une valeur d'absorbance de la solution de colorant dans l'isopropanol est mesurée.

11. La méthode selon l'une quelconque des revendications de 1 à 10, dans laquelle, lors de l'étape d'évaluation, la viabilité des cellules humaines est évaluée comme moyenne de la viabilité évaluée pour chaque morceau de tissu.

12. La méthode selon l'une quelconque des revendications de 1 à 11, dans laquelle chaque morceau de tissu a une largeur maximale comprise entre 1 mm et 10 mm, de préférence entre 2 mm et 4 mm.

13. La méthode selon l'une quelconque des revendications de 1 à 12, comprenant également, avant l'étape de sélection, une étape de préparation des morceaux de tissu qui prévoit à son tour les étapes suivantes :
explanter un globe oculaire chez un sujet décédé ;
conserver le globe oculaire explanté dans un milieu de culture DMEM/F-12 à une température nominale de +4°C ;
extraire la partie optique de la rétine du globe oculaire dans les soixante-douze heures à compter de la mort, de préférence dans les quarante-huit heures à compter de la mort ;
obtenir des morceaux de tissu rétinien à partir de la partie optique de la rétine par poinçonnage/carottage sur toute l'épaisseur, avec une plage de diamètre comprise entre 2 mm et 10 mm, de préférence avec une plage de diamètre allant de 2,5 mm à 3,5 mm.

14. Une méthode pour l'évaluation de la cytotoxicité d'une substance, dans laquelle une méthode selon l'une quelconque des revendications de 1 à 13 est effectuée plusieurs fois pour la même substance à tester, en utilisant à chaque fois une seule partie optique d'une rétine humaine différente, et dans laquelle la substance à tester est considérée comme étant cytotoxique si tel est le résultat de l'étape de détermination d'au moins une des fois pour lesquelles la méthode selon l'une quelconque des revendications de 1 à 13 est effectuée.
